# EUROPÄISCHE PATENTSCHRIFT

(11) **EP 3 793 748 B1**
(45) Veröffentlichungstag und Bekanntmachung des Hinweises auf die Patenterteilung: **05.03.2025**
(21) Anmeldenummer: 19732879.2
(22) Anmeldetag: 16.05.2019
(51) Int. Cl.: B05C 17/005, A61M 5/24, B67B 7/92

(54) **APPLIKATOR**
APPLICATOR
APPLICATEUR

(30) Priorität: 16.05.2018 DE 102018111805
(43) Veröffentlichungstag der Anmeldung: 24.03.2021
(73) Patentinhaber: Seitz Hiemer Sogaro GBR, 91602 Dürrwangen (DE)
(72) Erfinder: HIEMER, Andreas, 22926 Ahrensburg (DE); SÒGARO, Alberto C., 61348 Bad Homburg (DE); SEITZ, Sebastian, 91602 Dürrwangen (DE)
(74) Vertreter: advotec.
(86) Internationale Anmeldenummer: PCT/DE2019/100443
(87) Internationale Veröffentlichungsnummer: WO 2019/219130

(56) Entgegenhaltungen:
- CA-A1- 2 984 531
- US-A- 3 739 947
- US-A1- 2009 171 361
- US-A1- 2014 124 534

## Beschreibung

Die Erfindung betrifft einen Applikator zum Applizieren eines fließfähigen Stoffes, umfassend die Merkmale des Oberbegriffs des Anspruchs 1.

Derartige Applikatoren sind aus der Praxis bekannt und dienen beispielsweise zum Aufbringen eines Medikaments oder eines kosmetischen Stoffes auf die Haut eines Menschen oder Tieres, oder auch zum Applizieren eines sonstigen Stoffes, wie eines Klebstoffs. Der Applikator kann mit einer zerbrechbaren Ampulle versehen sein, in der der fließfähige Stoff vorgehalten wird. Ein präzises Dosieren des fließfähigen Stoffes ist mit den bisher bekannten Applikatoren häufig nicht möglich.

Ein Applikator mit den Merkmalen des Oberbegriffs des Patentanspruchs 1 ist aus der Druckschrift US 3,739,947 bekannt und umfasst eine Außenhülse mit einer zylindrischen Innenfläche und einem Aufnahmeraum für einen zu applizierenden fließfähigen Stoff. In der Außenhülse ist eine Innenhülse verschiebbar, in der eine brechbare Ampulle angeordnet ist, die den fließfähigen Stoff enthält. Die Innenhülse weist Öffnungen auf, die einen Innenraum der Innenhülse mit dem Aufnahmeraum der Außenhülse verbinden. Durch die Betätigung eines Stößels kann die Ampulle zerbrochen werden.

Aus der Druckschrift US 2014/0124534 A1 ist eine spritzenartige Applikationsvorrichtung bekannt. Diese Vorrichtung umfasst einen Drücker, in dem eine zerbrechbare Ampulle aufgenommen ist und der in einer Außenhülse geführt ist.

Aus der Druckschrift US 2009/0171361 A1 ist eine Vorrichtung zum Anrühren und Applizieren eines Knochenzements bekannt. Die Vorrichtung umfasst eine Außenhülse und eine in der Außenhülse geführte Innenhülse. In der Innenhülse ist eine zerbrechbare Ampulle angeordnet, die eine Knochenzementkomponente aufnimmt.

Der Erfindung liegt die Aufgabe zugrunde, einen Applikator zu schaffen, mit dem der in der zerbrechbaren Ampulle vorgehaltene fließfähige Stoff in definierter Weise zu einer Austragöffnung des Applikators förderbar ist.

Diese Aufgabe ist erfindungsgemäß durch den Applikator mit den Merkmalen des Anspruchs 1 gelöst.

Erfindungsgemäß wird also ein Applikator vorgeschlagen, der eine Außenhülse mit einer zylindrischen Innenfläche und einem Aufnahmeraum für einen zu applizierenden fließfähigen Stoff und eine in der Außenhülse verschiebbare Innenhülse umfasst, in der eine brechbare, den fließfähigen Stoff enthaltende Ampulle angeordnet ist und die eine Öffnung aufweist, die einen Innenraum der Innenhülse mit dem Aufnahmeraum der Außenhülse verbindet. Zudem ist eine Aktivierungseinrichtung vorgesehen, bei deren Betätigung die Ampulle bricht. Die Ampulle weist zwei abbrechbare Spitzen auf.

Die Ampulle, die eine Ampulle mit zwei abbrechbaren Spitzen ist, wird dadurch gebrochen, dass eine Rampe der Aktivierungseinrichtung auf die Ampulle bzw. auf eine der Spitzen der Ampulle auffährt.

In der Innenhülse ist eine weitere Rampe ausgebildet, auf die die Ampulle bzw. eine Spitze der Ampulle bei einer Betätigung der Aktivierungseinrichtung auffährt, so dass diese Spitze abgebrochen wird und der zu applizierende fließfähige Stoff aus der Ampulle ausfließen kann. Der Applikator nach der Erfindung arbeitet mithin derart, dass zunächst die Ampulle zerbrochen wird, so dass der fließfähige Stoff frei in dem Innenraum der Innenhülse enthalten ist. Über die Öffnung kann der fließfähige Stoff dann aus dem Innenraum der Innenhülse in den Aufnahmeraum der Außenhülse fließen und über eine Austragöffnung des Applikators dem Anwendungsfall entsprechend appliziert werden.

Der Applikator nach der Erfindung dient beispielsweise zum Applizieren eines Medikaments oder eines kosmetischen Stoffes auf die Haut eines menschlichen oder tierischen Körpers. Auch die Benutzung des Applikators im Dentalbereich ist denkbar. Der Applikator kann ferner auch zur Applikation von sonstigen Stoffen, wie Klebstoffen oder dergleichen eingesetzt werden.

Bei einer bevorzugten Ausführungsform des Applikators nach der Erfindung ist die Aktivierungseinrichtung aus einem stirnseitigen Einsatz der Innenhülse gebildet, der bei seiner Betätigung einen Versatz in die Innenhülse erfährt und die Ampulle zerbricht. Beispielsweise ist die Innenhülse als rohrartiges Element mit einer zylindrischen Innenfläche ausgebildet, an der die Aktivierungseinrichtung verlagerbar ist. Insbesondere kann der die Aktivierungseinrichtung bildende stirnseitige Einsatz nach Art eines Druckknopfes ausgebildet sein, der manuell in die Innenhülse gedrückt wird, wodurch die Ampulle zerbrochen wird.

Um den fließfähigen Stoff nach dem Brechen der Ampulle in den Aufnahmeraum zu bringen, kann die Innenhülse aus der Außenhülse gezogen werden. Dadurch wird in dem Aufnahmeraum ein Unterdruck erzeugt, wodurch der fließfähige Stoff aus dem Innenraum der Innenhülse über die Öffnung in den Aufnahmeraum fließt. Eine Abflussöffnung des Aufnahmeraums sollte hierbei natürlich geschlossen sein, was mittels eines Verschlusses oder auch mittels eines Rückschlagventils realisierbar ist.

Bei einer speziellen Ausführungsform des Applikators nach der Erfindung dient die Innenhülse als Kolben zum Ausbringen des fließfähigen Stoffes über eine Austragöffnung. Dies bedeutet, dass die Innenhülse zum Applizieren des fließfähigen Stoffs in die Außenhülse verlagert wird, wenn der fließfähige Stoff in dem Aufnahmeraum der Außenhülse angeordnet ist.

Bei einer speziellen Ausführungsform des Applikators nach der Erfindung ist ein Einwegeventil vorgesehen, das ein Zurückströmen des fließfähigen Stoffes aus dem Aufnahmeraum in die Innenhülse verhindert. Diese Ausführungsform ermöglicht es auch, den fließfähigen Stoff aus dem Innenraum der Innenhülse dadurch in den Aufnahmeraum der Außenhülse zu bringen, dass die Innenhülse aus der Außenhülse gezogen wird und so in der Außenhülse bzw. dem Aufnahmeraum der Außenhülse ein Unterdruck erzeugt wird, der den fließfähigen Stoff über die Öffnung aus der Innenhülse in den Aufnahmeraum der Außenhülse zieht.

Bei einer kostengünstig umsetzbaren Ausführungsform des Applikators nach der Erfindung ist das Einwegeventil aus einem gummielastischen Teller gebildet, der an einer Stirnseite der Innenhülse befestigt ist und mit der Öffnung der Innenhülse zusammenwirkt. Der gummielastische Teller bildet bei einem Applizieren des Stoffes eine Kolbenfläche, mittels der der fließfähige Stoff aus dem Aufnahmeraum gedrückt wird.

Um die Aktivierungseinrichtung in definierter Weise drücken zu können, weist die Innenhülse bei einer speziellen Ausführungsform des Applikators nach der Erfindung eine Führungsbahn für die Aktivierungseinrichtung auf. Die Führungsbahn kann schlitzartig ausgebildet sein. Korrespondierend kann an der Aktivierungseinrichtung ein Noppen bzw. Vorsprung ausgebildet sein, der in der Führungsbahn angeordnet ist und die Aktivierungseinrichtung in der Innenhülse hält.

Zur Erleichterung der Benutzung des Applikators nach der Erfindung kann die Innenhülse einen Bund oder Laschen zum Greifen durch einen Benutzer aufweisen. Entsprechend kann die Außenhülse einen Bund oder Laschen zur Anlage von Fingern eines Benutzers haben.

Eine weitere spezielle Ausführungsform des Applikators nach der Erfindung weist einen zweiten Aufnahmeraum auf, der von dem Aufnahmeraum über eine Öffnung und ein zweites Rückschlagventil getrennt ist. Dieser Aufnahmeraum ist ein Applikationsraum, in welcher ein Kolben zum Applizieren des fließfähigen Stoffes verschiebbar gelagert sein kann.

Zur Betätigung des Applikators nach der Erfindung kann eine Betätigungseinrichtung vorgesehen sein, die den Kolben gegenüber der Außenhülse verstellt. Beispielsweise greift die Außenhülse über ein Gewinde in ein Gewinde ein, welches in Axialrichtung ortsfest gegenüber einem Applikatorgehäuse angeordnet ist. Bei einer Betätigung der Betätigungseinrichtung wird das axial gegenüber dem Applikatorgehäuse ortsfeste Gewinde gedreht, so dass die Außenhülse verlagert und der Kolben in den Applikationsraum geschoben wird. Dadurch wird der in dem Applikationsraum enthaltene fließfähige Stoff über eine Austragöffnung ausgetragen.

Weitere Vorteile und vorteilhafte Ausgestaltungen des Gegenstandes der Erfindung ergeben sich aus der Beschreibung, der Zeichnung und den Patentansprüchen.

Ausführungsbeispiele eines Applikators nach der Erfindung sind in der Zeichnung schematisch vereinfacht dargestellt und werden in der nachfolgenden Beschreibung näher erläutert. Es zeigt:
- Figur 1: eine perspektivische Darstellung eines Applikators nach der Erfindung;
- Figur 2: eine Seitenansicht des Applikators nach Figur 1;
- Figur 3: den Applikator nach Figur 1 im zerlegten Zustand;
- Figur 4: einen Längsschnitt durch den Applikator entlang der Linie A-A in Figur 2;
- Figur 5: einen Figur 4 entsprechenden Längsschnitt des Applikators, jedoch nach Betätigung einer Aktivierungseinrichtung;
- Figur 6: einen Figur 5 entsprechenden Längsschnitt des Applikators, jedoch im Applikationszustand;
- Figur 7: eine perspektivische Ansicht einer zerbrechbaren Ampulle des Applikators;
- Figur 8: eine erste perspektivische Darstellung einer zweiten Ausführungsform eines Applikators nach der Erfindung;
- Figur 9: eine zweite perspektivische Darstellung des Applikators nach Figur 8;
- Figur 10: einen Überblick über die Einzelteile des Applikators nach den Figuren 8 und 9;
- Figur 11: einen Längsschnitt durch den Applikator nach den Figuren 8 und 9 in dessen Lieferzustand;
- Figur 12: einen Figur 11 entsprechenden Längsschnitt durch den Applikator, jedoch nach Betätigung einer Aktivierungseinrichtung;
- Figur 13: ebenfalls einen Figur 11 entsprechenden Längsschnitt durch den Applikator, jedoch nach dem Befüllen eines Aufnahmeraums des Applikators;
- Figur 14: einen Schnitt durch den Applikator entlang der Linie XIV-XIV in Figur 13;
- Figur 15: eine perspektivische Darstellung einer dritten Ausführungsform eines Applikators nach der Erfindung;
- Figur 16: eine weitere perspektivische Darstellung des Applikators nach Figur 15;
- Figur 17: eine perspektivische Darstellung der Einzelteile des Applikators nach den Figuren 15 und 16;
- Figur 18: einen Längsschnitt durch den Applikator nach den Figuren 15 und 16 in dessen Lieferzustand;
- Figur 19: einen Figur 18 entsprechenden Längsschnitt durch den Applikator, jedoch nach Betätigung einer Aktivierungseinrichtung;
- Figur 20: einen Figur 18 entsprechenden Längsschnitt durch den Applikator, jedoch nach dem Befüllen eines Aufnahmeraums des Applikators;
- Figur 21: einen Schnitt durch den Applikator nach Figur 20 entlang der Linie XXI-XXI in Figur 20; und
- Figur 22: eine perspektivische Ansicht einer vierten Ausführungsform eines Applikators nach der Erfindung.

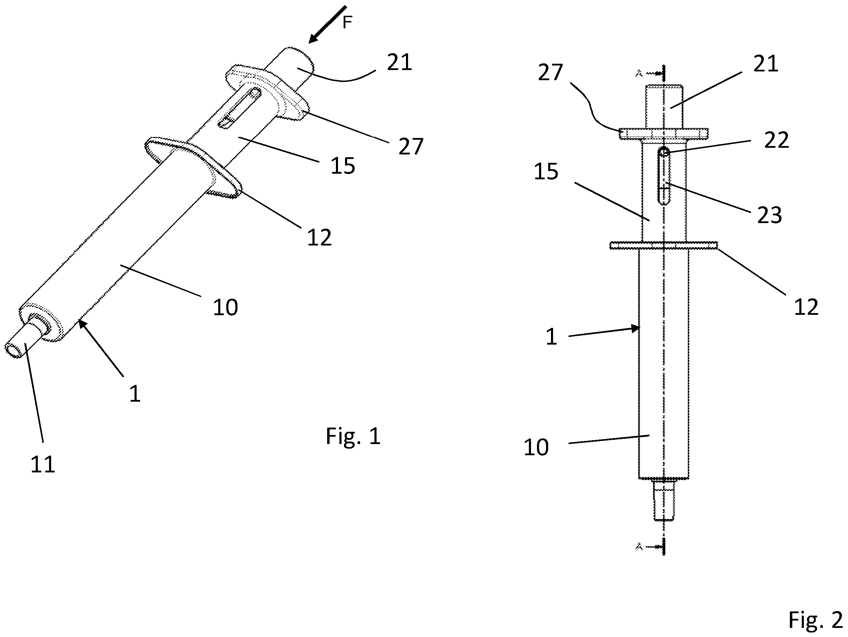

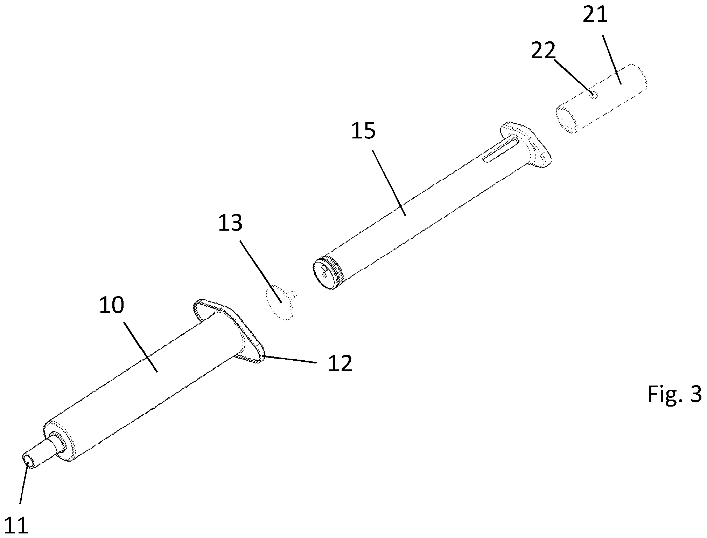

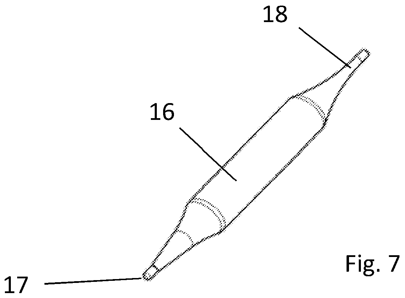

In den Figuren 1 bis 7 ist ein Applikator 1 dargestellt, der zur Applikation eines fließfähigen Stoffes, wie eines Medikaments, eines kosmetischen Stoffes oder eines Klebstoffs dient. Der Applikator 1 umfasst eine Außenhülse 10, an der stirnseitig eine Austragöffnung 11 und an dem der Austragöffnung abgewandten Ende ein Bund 12 ausgebildet ist, der als Greifhilfe dient. Die Außenhülse 10 hat eine zylindrische Innenfläche, an der eine Innenhülse 15 geführt ist. Die Innenhülse 15 nimmt in dem in den Figuren 1, 2 und 4 dargestellten Lieferzustand des Applikators 1 eine zerbrechbare Ampulle 16 aus Glas auf, die zwei abbrechbare Spitzen 17 und 18 aufweist.

An ihrer der Austragöffnung 11 zugewandten Öffnung weist die Innenhülse 15 eine Öffnung 20 auf, welche mit einem Rückschlagventil 13 versehen ist, das aus einem pilzartigen Gummielement besteht, dessen Kopf das Dichtelement für die Öffnung 20 bildet und an der Stirnseite der Innenhülse 15 anliegt. Ein Fuß 14 des Rückschlagventils 13 ist in ein stirnseitiges Loch der Innenhülse 15 eingesteckt und hält das Rückschlagventil 13 so an der Innenhülse 15. Zudem hat die Innenhülse 15 an ihrem der Öffnung 20 abgewandten Ende einen Bund 27, der als Greifhilfe dient.

Des Weiteren weist der Applikator 1 eine Aktivierungseinrichtung 21 auf, die aus einem stirnseitigen, drückerartigen Einsatz der Innenhülse 15 besteht und an einer zylindrischen Innenseite der Innenhülse 15 verlagerbar ist. An seinem Umfang hat die Aktivierungseinrichtung zwei Noppen 22, die um 180° zueinander versetzt sind und jeweils in eine Führungsbahn 23 eingreifen, die an der Innenhülse 15 ausgebildet ist und sich in Längsrichtung des Applikators 1 erstreckt.

Die drückerartige Aktivierungseinrichtung 21 hat an ihrer Innenseite eine Rampe 24, welche bei der Betätigung der Aktivierungseinrichtung 21 auf die Spitze 18 der Ampulle 16 auffährt und diese abbricht. Zudem ist angrenzend an die Öffnung 20 in der Innenhülse 15 eine Rampe 25 ausgebildet, welche mit der Spitze 17 der Ampulle 16 zusammenwirkt und dazu dient, bei einer Betätigung der Aktivierungseinrichtung 21 die Spitze 17 abzubrechen und die Ampulle 16 zu öffnen. Des Weiteren weist die Innenhülse 15 einen Innenraum 26 auf, welcher die Ampulle 16 aufnimmt und über die Öffnung 20 mit einem Aufnahmeraum 28 verbunden ist, der in der Außenhülse 10 zwischen der Stirnseite der Innenhülse 15 bzw. dem Rückschlagventil 13 einerseits und der Austragöffnung 11 andererseits angeordnet ist.

Der in den Figuren 1 bis 7 dargestellte Applikator 1 arbeitet in nachfolgend beschriebener Weise.

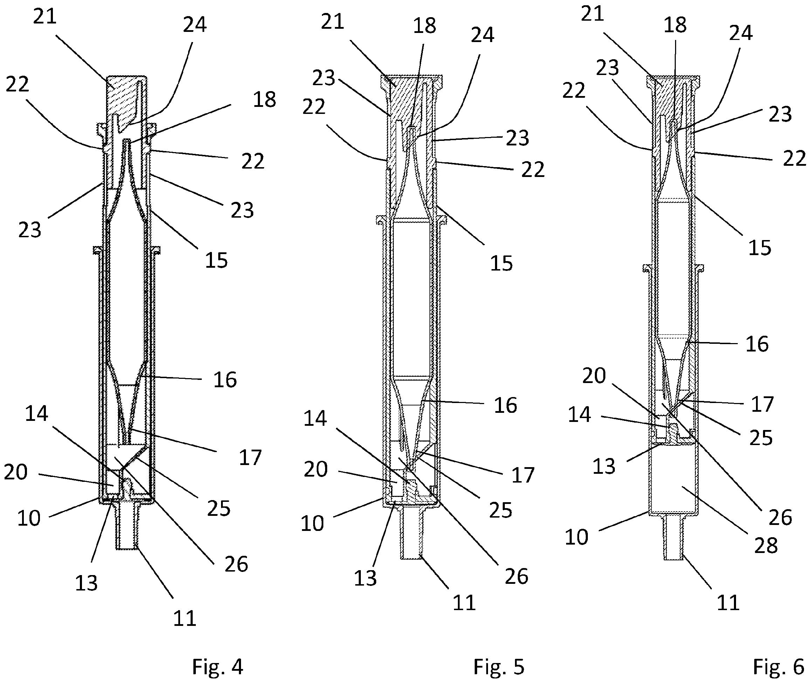

Ausgehend von dem in den Figuren 1, 2 und 4 dargestellten Lieferzustand wird bei einer Verwendung des Applikators 1 zunächst die drückerartige Aktivierungseinrichtung 21 gemäß Figur 5 in die Innenhülse 15 gedrückt, wodurch die Rampe 24 auf die Spitze 18 der Ampulle 16 auffährt und die Ampulle 16 mit Ihrer Spitze 17 gegen die Rampe 25 der Innenhülse 15 gedrückt wird. Dadurch werden die beiden Spitzen 17 und 18 abgebrochen und der in der Ampulle 16 enthaltene fließfähige Stoff kann in den Innenraum 26 der Innenhülse 15 fließen. In einem nächsten Schritt wird die in der Außenhülse 10 angeordnete Innenhülse in der der Austragöffnung 11 abgewandten Richtung ein Stück aus der Außenhülse 10 gezogen, wobei die Austragöffnung 11 durch einen Deckel, ein Rückschlagventil oder dergleichen geschlossen bleibt. Dadurch vergrößert sich der Aufnahmeraum 28 zwischen der Stirnseite der Innenhülse 15 und der Austragöffnung 11, wobei aufgrund des entstehenden Unterdrucks in dem Aufnahmeraum 28 der in der Innenhülse enthaltene fließfähige Stoff durch die Öffnung 20, die mit dem Rückschlag- bzw. Einwegeventil versehen ist, in den Aufnahmeraum 28 gefördert wird. Der Applikator 1 ist damit in einem Applikationszustand. Durch Ausüben eines Druckes F auf die hintere von der Aktivierungseinrichtung 21 gebildete Stirnseite der als Kolben dienenden Innenhülse 15 kann nun die in dem Aufnahmeraum 28 enthaltene Flüssigkeit über die dann geöffnete Austragöffnung 11, die ebenfalls mit einem Einwegeventil bzw. Rückschlagventil versehen sein kann, aus dem Applikator 1 gefördert und appliziert werden.

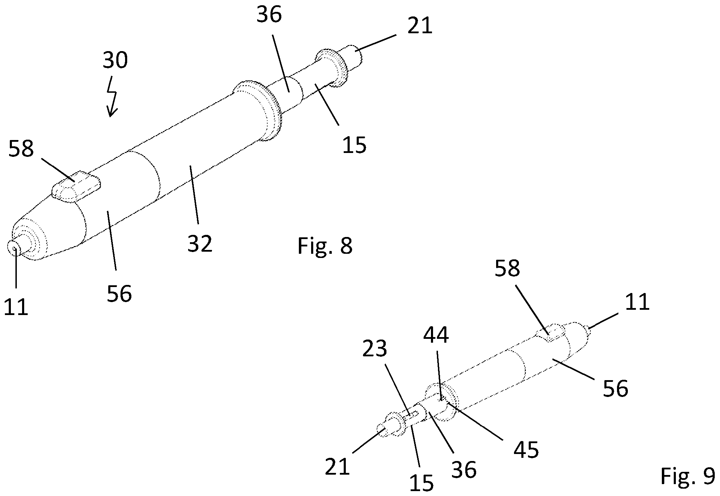

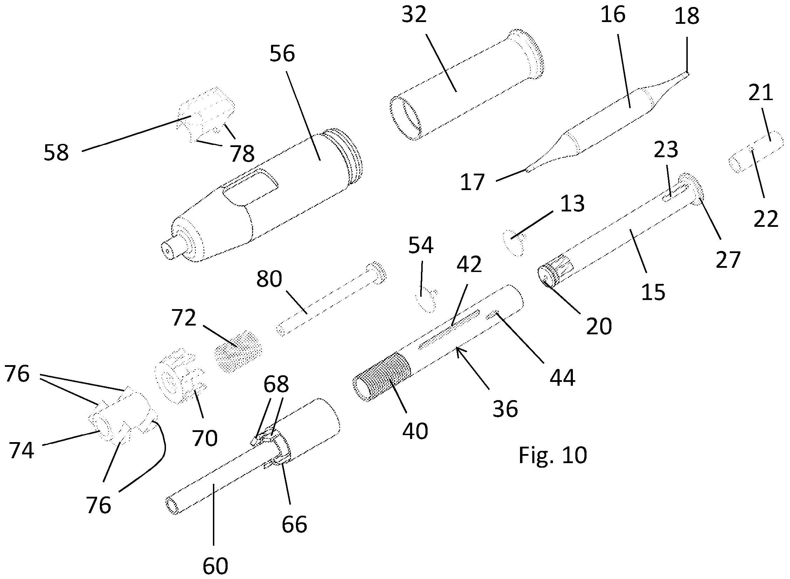

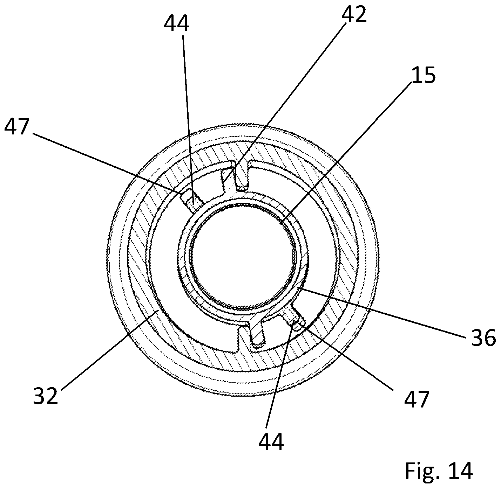

In den Figuren 8 bis 14 ist ein Applikator 30 dargestellt, der ebenfalls zum Applizieren eines fließfähigen Stoffes über eine Austragöffnung 11 dient. Der Applikator 30 umfasst ein Applikatorgehäuse 32, das eine im Wesentlichen zylindrische Aufnahme 34 aufweist, in welcher eine Außenhülse 36 angeordnet ist. Die Außenhülse 36 umfasst eine zylindrische Umfangswand 38, die in einem Endbereich mit einem Außengewinde 40 versehen ist. Zudem sind an der Umfangswand 38 Führungsrippen 42 ausgebildet, die sich in axialer Richtung erstrecken und an der Innenseite des Applikatorgehäuses 32 geführt sind. In Umfangsrichtung versetzt sind an der Umfangswand 38 der Außenhülse 36 zwei Anschlagrippen 44 ausgebildet, welche je nach Zustand mit der Stirnseite 46 oder einer Führungsnut 47 des Applikatorgehäuses 32 zusammenwirken und beim Applizieren des fließfähigen Stoffes als Verdrehsicherung der Aufnahmehülse 36 dienen

Die Außenhülse 36 umfasst eine Trennwand 48, in der ein Verbindungskanal 50 ausgebildet ist, der einen Aufnahmeraum 28, der der Austragöffnung 11 abgewandt ist, und einen Aufnahmeraum 52, der der Austragöffnung 11 zugewandt ist, miteinander verbindet. In den Aufnahmeraum 28 der Außenhülse 36 greift eine Innenhülse 15 ein, welche entsprechend der Innenhülse der Ausführungsform nach den Figuren 1 bis 7 ausgebildet ist, weswegen an dieser Stelle auf die Beschreibung der Innenhülse 15 in Verbindung mit dieser Ausführungsform verwiesen wird. Die Innenhülse 15 nimmt also eine zerbrechbare Ampulle 16 auf, die zwei Spitzen 17 und 18 aufweist, die abbrechbar sind. Zudem ist in die der Austragöffnung abgewandte Stirnseite der Innenhülse 15 eine Aktivierungseinrichtung 21 in Form eines drückerartigen Einsatzes eingesetzt, der an seiner Innenseite eine Rampe 24 aufweist. Ferner ist an der der Trennwand 48 der Außenhülse 36 zugewandten Stirnseite ein Rückschlagventil 13 angeordnet, das ebenfalls entsprechend dem Rückschlagventil der Ausführungsform nach den Figuren 1 bis 7 ausgebildet ist.

Der Verbindungskanal 50, der in der Trennwand 48 der Außenhülse 36 ausgebildet ist, wirkt mit einem weiteren Rückschlagventil 54 zusammen, das entsprechend dem Rückschlagventil 13 aus einem pilzartigen Gummielement besteht, dessen Fuß in einem Loch der Trennwand 48 gehalten ist.

Das Applikatorgehäuse 32 hat einen frontseitigen Gehäuseaufsatz 56, der mit einem Drücker 58 versehen ist, der bezogen auf die Achse des Applikators 30 gegenüber dem Gehäuseaufsatz 56 in radialer Richtung verlagerbar ist. An dem Gehäuseaufsatz 56 ist die Austragöffnung 11 ausgebildet. Zudem nimmt der Gehäuseaufsatz 56 ein Applikationsrohr 60 auf, das sich mit seiner der Innenhülse 15 abgewandten Stirnseite an dem Gehäuseaufsatz 56 abstützt und das eine Erweiterung 62 hat, an der ein Innengewinde 64 ausgebildet ist, das mit dem Außengewinde 40 der Außenhülse 36 in Eingriff steht. An einer der Innenhülse 15 abgewandten Stufe 66 der Erweiterung 62 sind Stellrampen 68 ausgebildet, die nach Art eines Kugelschreiberantriebs mit einem Gegenrampen aufweisenden Stellkranz 70 zusammenwirken, der drehbar auf dem Applikationsrohr 60 angeordnet ist. Zwischen dem Stellkranz 70 und der Erweiterung 62 des Applikationsrohrs 60 ist eine Rückstellfeder 72 eingespannt. Auf dem Applikationsrohr 60 ist ein Gleiter 74 verschiebbar gelagert, der über Stellrampen 76 mit Gegenrampen 78 zusammenwirkt, die an dem Drücker 58 angeordnet sind. Durch Zusammenwirken der Stellrampen 76 mit den Gegenrampen 78 wird der Gleiter 74 beim Drücken des Drückers 58 in axialer Richtung des Applikators 30 gegen den Stellkranz 70 verfahren, so dass dieser über seine Gegenrampen mit den Stellrampen 68 zusammenwirkt, wodurch das Applikationsrohr 60 gedreht und das Außengewinde 40 der Außenhülse 36 in das Innengewinde 64 des Applikationsrohrs 60 gedreht wird.

Das Applikationsrohr 60 ist von einem Kolbenrohr 80 durchgriffen, dessen eine Stirnseite sich im Bereich der Austragöffnung 11 an dem Gehäuseaufsatz 56 abstützt und dessen andere Stirnseite von einem Ringbund 82 gebildet ist, der eine Kolbenfläche bildet.

Der in den Figuren 8 bis 14 dargestellte Applikator 30 arbeitet in nachfolgend beschriebener Weise.

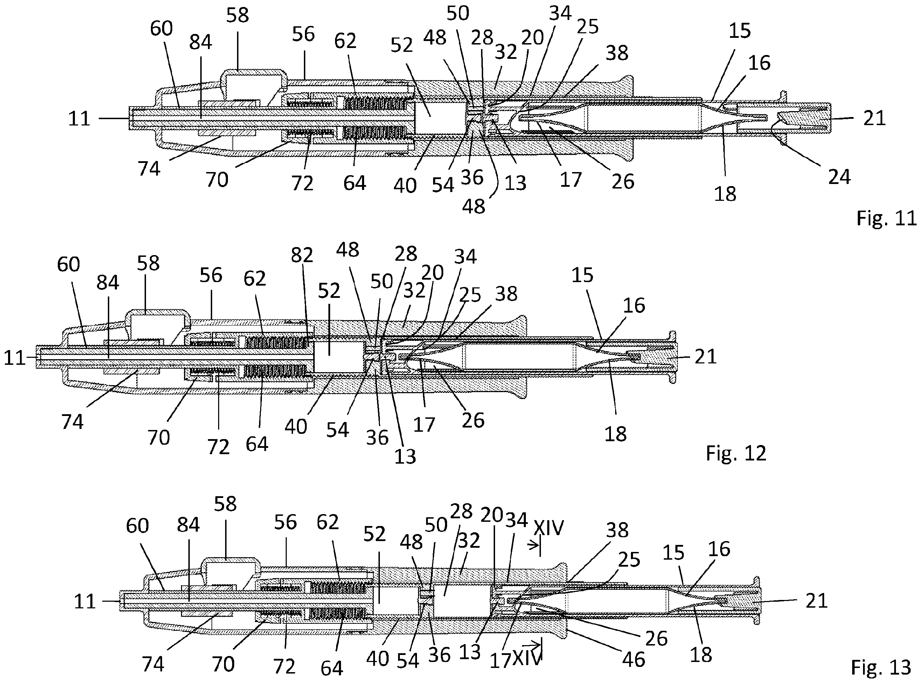

Ausgehend von dem in Figur 11 dargestellten Lieferzustand wird zunächst die Aktivierungseinrichtung 21 betätigt, so dass die Spitzen 17 und 18 der Ampulle 16 abgebrochen und die in der Ampulle 16 vorgehaltene Flüssigkeit in den Innenraum 26 der Innenhülse 15 fließen kann. Anschließend wird die Innenhülse 15 ein Stück aus der Außenhülse 36 gezogen, wodurch zwischen der Trennwand 48 und der Stirnwand der Innenhülse 15 der Aufnahmeraum 28 vergrößert wird und durch das resultierende Vakuum die Flüssigkeit aus dem Innenraum 26 der Innenhülse 15 in den Aufnahmeraum 28 gezogen wird (vgl. Figur 13). Anschließend wird die Einheit aus der Innenhülse 15 und der Aktivierungseinrichtung 21 nach Art eines Kolbens in die Außenhülse 36 gedrückt, wodurch die Flüssigkeit aus dem Aufnahmeraum 28 über den Verbindungskanal 50 in den Aufnahmeraum 52 gedrückt wird. Dann kann die Außenhülse 36 gegenüber dem Applikatorgehäuse 32 verdreht werden, wodurch die Anschlagrippen 44 freigegeben und in die Führungsnuten 47 des Applikatorgehäuses 32 eintauchen können. Nun ist der Applikator 30 aktiviert. Durch Drücken des Drückers 58 wird das Applikationsrohr 60 in der oben beschriebenen Weise gedreht, so dass die Außenhülse 36 in die Erweiterung 62 des Applikationsrohrs 60 eingedreht wird. Dadurch verdrängt das Kolbenrohr 80 die Flüssigkeit aus dem Aufnahmeraum 52 durch den Kolbenkanal 84 zu der Austragöffnung 11, über die die Flüssigkeit appliziert werden kann.

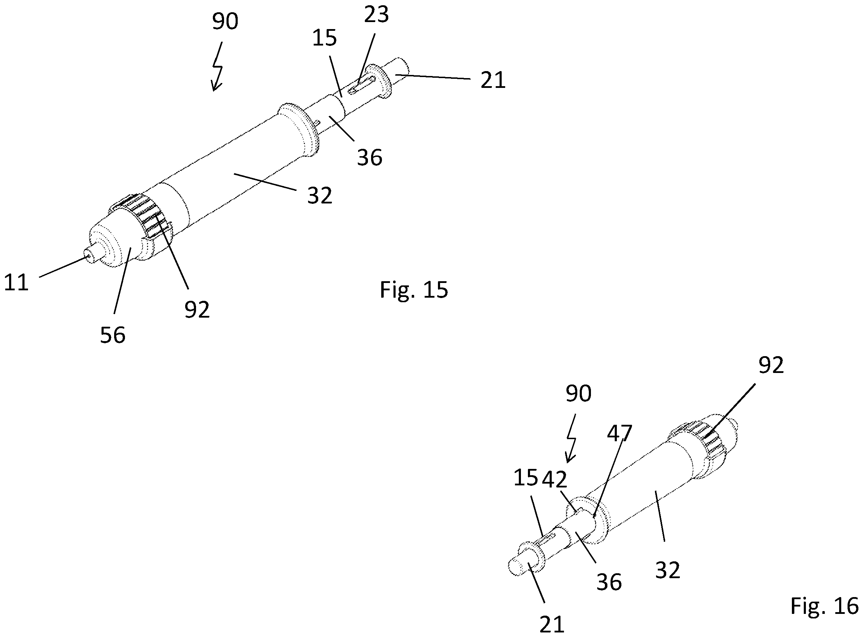

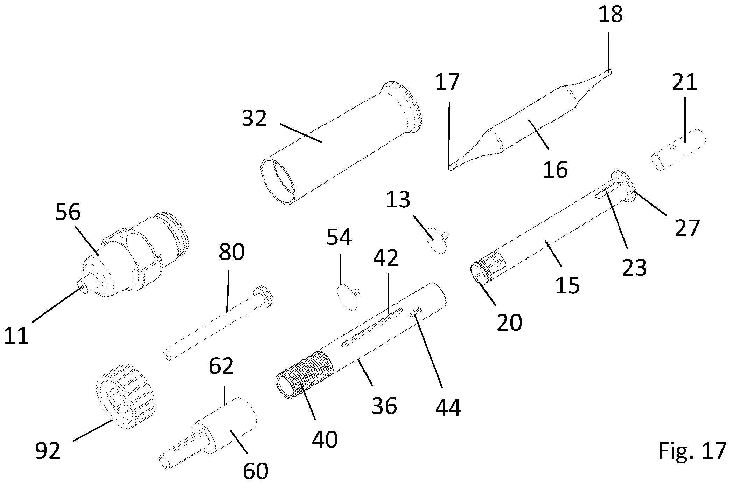

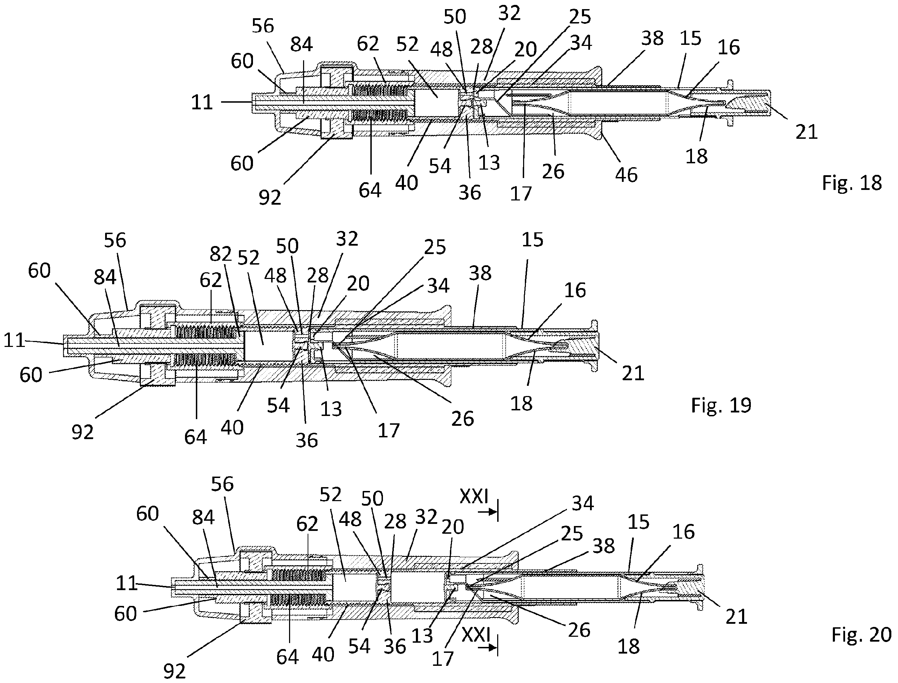

In den Figuren 15 bis 21 ist ein Applikator 90 dargestellt, der weitgehend demjenigen nach den Figuren 8 bis 14 entspricht, sich von diesem aber dadurch unterscheidet, dass er ein Applikationsrohr 60 aufweist, das mittels eines in Umfangsrichtung des Applikators 90 drehbaren Stellring 92 betätigbar ist. Durch Betätigung des Stellrings 92 wird das Applikationsrohr 60, das von dem Kolbenrohr 80 durchgriffen ist, gedreht, so dass das Außengewinde 40 der Außenhülse 36 in das Innengewinde 64 der Erweiterung 62 des Applikationsrohrs 60 eingedreht wird. Das Kolbenrohr 80 verdrängt dann die in dem Aufnahmeraum 52 der Außenhülse 36 enthaltene Flüssigkeit zu der Austragöffnung 11.

Im Übrigen entspricht der Applikator 90 demjenigen nach den Figuren 8 bis 14.

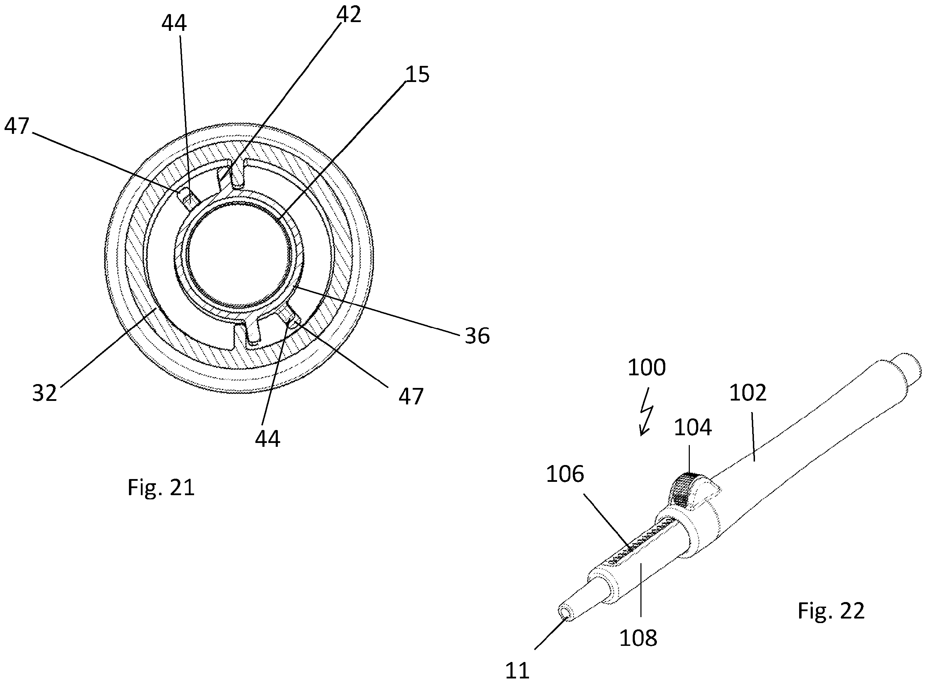

In Figur 22 ist ein Applikator 100 dargestellt, der weitgehend demjenigen nach den Figuren 15 bis 21 entspricht, sich von diesem aber dadurch unterscheidet, dass er ein Applikatorgehäuse 102 aufweist, das mit einem Stellrad 104 versehen ist, welches mit einer Axialverzahnung 106 zusammenwirkt, die an einem Applikationsrohr 108 ausgebildet ist, das gleitend an einer Außenhülse geführt ist, die entsprechend der Außenhülse der Ausführungsform nach den Figuren 15 bis 21 ausgebildet ist, aber kein Außengewinde aufweist. Durch Betätigung des Stellrads 104 wird das Applikationsrohr 108 zusammen mit einem von dem Applikationsrohr 108 aufgenommenen Kolbenrohr 80 zur Applikation der Flüssigkeit über eine Austragöffnung 11 an der Außenhülse verfahren.

### Bezugszeichenliste

- 1: Applikator
- 10: Außenhülse
- 11: Austragöffnung
- 12: Bund
- 13: Rückschlagventil
- 14: Fuß
- 15: Innenhülse
- 16: Ampulle
- 17: Spitze
- 18: Spitze
- 20: Öffnung
- 21: Aktivierungseinrichtung
- 22: Noppen
- 23: Führungsbahn
- 24: Rampe
- 25: Rampe
- 26: Innenraum
- 27: Bund
- 28: Aufnahmeraum
- 30: Applikator
- 32: Applikatorgehäuse
- 34: Aufnahme
- 36: Außenhülse
- 38: Umfangswand
- 40: Außengewinde
- 42: Führungsrippen
- 44: Anschlagrippen
- 46: Stirnseite
- 47: Führungsnuten
- 48: Trennwand
- 50: Verbindungskanal
- 52: Aufnahmeraum
- 54: Rückschlagventil
- 56: Gehäuseaufsatz
- 58: Drücker
- 60: Applikationsrohr
- 62: Erweiterung
- 64: Innengewinde
- 66: Stufe
- 68: Stellrampe
- 70: Stellkranz
- 72: Rückstellfeder
- 74: Gleiter
- 76: Stellrampe
- 78: Gegenrampe
- 80: Kolbenrohr
- 82: Ringbund
- 84: Kolbenkanal
- 90: Applikator
- 92: Stellring
- 100: Applikator
- 102: Applikatorgehäuse
- 104: Stellrad
- 106: Axialverzahnung
- 108: Applikationsrohr

## Patentansprüche

1. Applikator, umfassend eine Außenhülse (10, 36) mit einer zylindrischen Innenfläche und einem Aufnahmeraum (28) für einen zu applizierenden fließfähigen Stoff, eine brechbare Ampulle (16), die den fließfähigen Stoff enthält, und eine in der Außenhülse (10, 36) verschiebbare Innenhülse (15), in der die brechbare, den fließfähigen Stoff enthaltende Ampulle (16) angeordnet ist und die eine Öffnung (20) aufweist, die einen Innenraum (26) der Innenhülse (15) mit dem Aufnahmeraum (28) der Außenhülse (10, 36) verbindet, sowie eine Aktivierungseinrichtung (21), bei deren Betätigung die Ampulle (16) bricht, **dadurch gekennzeichnet, dass** die Ampulle (16) zwei abbrechbare Spitzen (17, 18) aufweist, wobei die Aktivierungseinrichtung (21) eine Rampe (24) aufweist, die bei der Betätigung auf die Ampulle (16) auffährt und in der Innenhülse (15) eine weitere Rampe (25) ausgebildet ist, auf die die Ampulle (16) bei einer Betätigung der Aktivierungseinrichtung (21) auffährt, so dass sie bricht.

2. Applikator nach Anspruch 1, **dadurch gekennzeichnet, dass** die Aktivierungseinrichtung (21) ein stirnseitiger Einsatz der Innenhülse (15) ist, der bei seiner Betätigung einen Versatz in die Innenhülse (15) erfährt und die Ampulle (16) zerbricht.

3. Applikator nach Anspruch 1 oder 2, **dadurch gekennzeichnet, dass** durch Verschieben der Innenhülse (15) in der Außenhülse (10, 36) ein Unterdruck in dem Aufnahmeraum (28) erzeugbar ist, so dass der fließfähige Stoff, der in dem Innenraum (26) der Innenhülse (15) enthalten ist, über die Öffnung (20) in den Aufnahmeraum (28) fließt.

4. Applikator nach einem der Ansprüche 1 bis 3, **dadurch gekennzeichnet, dass** die Innenhülse (15) als Kolben zum Ausbringen des fließfähigen Stoffes über eine Austragöffnung (11) dient.

5. Applikator nach einem der Ansprüche 1 bis 4, **gekennzeichnet durch** ein Rückschlagventil (13), das ein Zurückströmen des fließfähigen Stoffes aus dem Aufnahmeraum (28) in die Innenhülse (15) verhindert.

6. Applikator nach Anspruch 5, **dadurch gekennzeichnet, dass** das Rückschlagventil (13) aus einem gummielastischen Teller gebildet ist, der an einer Stirnseite der Innenhülse (15) befestigt ist und mit der Öffnung (20) der Innenhülse (15) zusammenwirkt.

7. Applikator nach einem der Ansprüche 1 bis 6, **dadurch gekennzeichnet, dass** die Innenhülse (15) eine Führungsbahn (23) für die Aktivierungseinrichtung (21) hat.

8. Applikator nach einem der Ansprüche 1 bis 7, **dadurch gekennzeichnet, dass** die Innenhülse (15) einen Bund (27) zum Greifen durch einen Benutzer hat.

9. Applikator nach einem der Ansprüche 1 bis 8, **dadurch gekennzeichnet, dass** die Außenhülse (10) einen Bund (12) zur Anlage von Fingern eines Benutzers hat.

## Claims

1. An applicator comprising an outer sleeve (10, 36) having a cylindrical inner surface and a receiving space (28) for a flowable substance to be applied, a frangible ampule (16) containing the flowable substance and an inner sleeve (15) which is displaceable in the outer sleeve (10, 36) and in which the frangible ampule (16) containing the flowable substance is disposed and which has an opening (20) connecting an interior (26) of the inner sleeve (15) to the receiving space (28) of the outer sleeve (10, 36), as well as comprising an activation means (21) upon the activation of which the ampule (16) breaks, **characterized in that** the ampule (16) has two tips (17, 18) which can be snapped off, the activation means (21) comprising a ramp (24) which ascends on the ampule (16) upon activation, another ramp (25) being formed in the inner sleeve (15), the ramp (25) ascending on the ampule (16) upon activation of the activation means (21) so that the ampule (16) breaks.

2. The applicator according to claim 1, **characterized in that** the activation means (21) is a frontal insert of the inner sleeve (15) which is displaced into the inner sleeve (15) upon activation and breaks the ampule (16).

3. The applicator according to any one of the claims 1 or 2, **characterized in that** a negative pressure is generable in the receiving space (28) as a consequence of displacing the inner sleeve (15) in the outer sleeve (10, 36) so that the flowable substance contained in the interior (26) of the inner sleeve (15) flows into the receiving space (28) via the opening (20).

4. The applicator according to any one of the claims 1 to 3, **characterized in that** the inner sleeve (15) serves as a piston for discharging the flowable substance via the discharge opening (11).

5. The applicator according to any one of the claims 1 to 4, **characterized by** a check valve (13) which prevents the flowable substance from flowing back into the inner sleeve (15) from the receiving space (28).

6. The applicator according to claim 5, **characterized in that** the check valve (13) is made of a rubbery-elastic plate which is fastened to a front side of the inner sleeve (15) and interacts with the opening (20) of the inner sleeve (15).

7. The applicator to any one of the claims 1 to 6, **characterized in that** the inner sleeve (15) has a guide track (23) for the activation means (21).

8. The applicator according to any one of the claims 1 to 7, **characterized in that** the inner sleeve (15) has a collar (27) for being gripped by a user.

9. The applicator according to any one of the claims 1 to 8, **characterized in that** the outer sleeve (10) has a collar (12) against which a user's fingers abut.

## Revendications

1. Applicateur comprenant une douille extérieure (10, 36) ayant une surface intérieure cylindrique et un espace de réceptacle (28) pour une substance fluide à appliquer, une ampoule (16) frangible contenant la substance fluide et une douille intérieure (15) qui est déplaçable dans la douille extérieure (10, 36) et dans laquelle est disposée l'ampoule (16) frangible contenant la substance fluide et qui a une ouverture (20) reliant un intérieur (26) de la douille intérieure (15) à l'espace de réceptacle (28) de la douille extérieure (10, 36), ainsi qu'ayant un moyen d'activation (21) sur l'activation duquel l'ampoule (16) se brise, **caractérisé en ce que** l'ampoule (16) a deux points (17, 18) qui peuvent être cassés, le moyen d'activation (21) ayant une rampe (24) qui monte sur l'ampoule (16) lors de l'activation, une autre rampe (25) étant formée dans la douille intérieure (15), la rampe (25) montant sur l'ampoule (16) lors de l'activation du moyen d'activation (21) de sorte que l'ampoule (16) se brise.

2. Applicateur selon la revendication 1, **caractérisé en ce que** le moyen d'activation (21) est un insert frontal de la douille intérieure (15) qui est déplacé dans la douille intérieure (15) lors de l'activation et casse l'ampoule (16).

3. Applicateur selon l'une quelconque des revendications 1 ou 2, **caractérisé en ce qu'**une pression négative est générée dans l'espace de réceptacle (28) en conséquence du déplacement de la douille intérieure (15) dans la douille extérieure (10, 36) de sorte que la substance fluide contenue à l'intérieur (26) de la douille intérieure (15) s'écoule dans l'espace de réceptacle (28) via l'ouverture (20).

4. Applicateur selon l'une quelconque des revendications 1 à 3, **caractérisé en ce que** la douille intérieure (15) sert de piston pour décharger la substance fluide par l'ouverture de décharge (11).

5. Applicateur selon l'une quelconque des revendications 1 à 4, **caractérisé par** un clapet anti-retour (13) qui empêche la substance fluide de refluer dans la douille intérieure (15) à partir de l'espace de réceptacle (28).

6. Applicateur selon la revendication 5, **caractérisé en ce que** le clapet anti-retour (13) est constitué d'une plaque élastique en caoutchouc qui est fixée à un côté avant de la douille intérieure (15) et interagit avec l'ouverture (20) de la douille intérieure (15).

7. Applicateur selon l'une quelconque des revendications 1 à 6, **caractérisé en ce que** la douille intérieure (15) a une piste de guidage (23) pour le moyen d'activation (21).

8. Applicateur selon l'une quelconque des revendications 1 à 7, **caractérisé en ce que** la douille intérieure (15) a une collerette (27) destinée à être saisie par un utilisateur.

9. Applicateur selon l'une quelconque des revendications 1 à 8, **caractérisé en ce que** la douille extérieure (10) a une collerette (12) contre laquelle viennent buter les doigts de l'utilisateur.
